# EUROPÄISCHE PATENTANMELDUNG

(11) **EP 0 549 975 A1**
(43) Veröffentlichungstag der Anmeldung: **07.07.1993**
(21) Anmeldenummer: 92121559.6
(22) Anmeldetag: 18.12.1992
(51) Int. Cl.: A23K 1/00, A23K 1/16, A61K 47/46

(54) **Arzneimittel- und Futtermittel-Vormischungen**

(30) Priorität: 21.12.1991 DE 4142633
(71) Anmelder: HOECHST AKTIENGESELLSCHAFT, 65926 Frankfurt am Main (DE)
(72) Erfinder: Blecher, Arnd, W-6365 Rosbach 1 (DE); Hoffmann, Rüdiger, W-6230 Frankfurt am Main 80 (DE); Hornkiewytsch, Theophil, Dr., W-6230 Frankfurt am Main 80 (DE)

(57) **Zusammenfassung**

Die Erfindung betrifft eine Wirkstoffzubereitung, die dadurch gekennzeichnet ist, daß sie einen oder mehrere Wirkstoffe (Arzneimittel- und Futterzusatzstoffe) und grobteilige Getreidekleie als Trägerstoff enthält. Bei dieser Wirkstoffzubereitung handelt es sich um Arzneimittel- und Futtermittelvormischungen.

Die Erfindung betrifft außerdem ein Verfahren zur Herstellung dieser Wirkstoffzubereitung, das dadurch gekennzeichnet ist, daß der Wirkstoff und der Trägerstoff innig miteinander vermengt werden.

## Beschreibung

### Arzneimittel- und Futtermittel-Vormischungen

In der Tierhaltung, insbesondere der Massentierhaltung, spielen Vormischungen zur Applikation von Arzneimitteln und Futterzusatzstoffen eine große Rolle. Vormischungen sind konzentrierte Mischungen des Arzneimittels bzw. des Futterzusatzstoffes mit einem oder mehreren Trägerstoffen. Sie werden vom Anwender vor der Verabreichung an die Tiere auf die Anwendungskonzentration abgemischt. Um während des Transportes und des Mischens beim Anwender Entmischungen und damit Inhomogenität in der Endmischung zu vermeiden, sind hohe Anforderungen an die physikalischen Eigenschaften der Vormischung zu stellen. Die Eigenschaften der Vormischung, vor allem Fließfähigkeit, Mischbarkeit, Entmischungsverhalten, Staubzahl und Neigung zum Verbacken unter Druckbelastung werden von dem oder den Trägerstoffen, die in der Vormischung in der Regel einen Anteil von mehr als 90 % haben, bestimmt. Der Auswahl des geeigneten Trägerstoffes kommt daher entscheidende Bedeutung für die Qualität der Vormischung zu.

Der Hersteller einer stabilen Vormischung wird versuchen, die unterschiedlichen Qualitätsanforderungen zu optimieren, wobei das Problem bestand, daß zum Beispiel durch die Zumischung grober Getreideteile zwar die Fließfähigkeiten verbessert werden, aber die Mischstabilität verschlechtert wird (IFF-Dienst Nr. 221, Institut für Futtermittelforschung, Braunschweig-Thune, Januar/Februar 1990).

Besteht der Trägerstoff nämlich aus Partikeln mit unterschiedlichen Korngrößen, so kann ein Entmischen stattfinden. Dieser Entmischungsprozeß wird umso mehr begünstigt, je größer der Unterschied in der Korngröße ist, da die kleinen Partikel die Möglichkeit haben, durch die Zwischenräume der großen Partikel hindurchzufallen. Die vorherrschende Lehrmeinung legte also nahe, Vormischungen aus Trägerstoffen und wirksamen Substanzen möglichst gleicher, kleiner Korngröße herzustellen. Auf diese Weise kann zwar die Tendenz zum Entmischen verhindert werden, es müssen dafür aber andere negative Eigenschaften, wie die Verminderung der Fließfähigkeit und ein Anstieg der Staubwerte, in Kauf genommen werden.

Überraschenderweise wurde festgestellt, daß grobkörnige Trägerstoffe keine größere Entmischung zeigen als feinkörnige.Im Vergleich zu Vormischungen mit feinkörnigen Trägestoffen weisen Vormischungen mit grobkörnigen Trägerstoffen deutlich günstigere physikalische Eigenschaften auf. Das Fließverhalten ist verbessert, eine meßbare Staubentwicklung tritt nicht auf und das Verbacken unter Druckbelastung wird reduziert.

Durch Einsatz grobteiliger Trägerstoffe können somit die beim Herstellen von Vormischungen auftretenden Probleme befriedigend gelöst werden.

Die Erfindung betrifft eine Wirkstoffzubereitung, die dadurch gekennzeichnet ist, daß sie einen oder mehrere Wirkstoffe (Arzneimittel- und Futterzusatzstoffe) und grobteilige Getreidekleie als Trägerstoff enthält. Bei dieser Wirkstoffzubereitung handelt es sich um Arzneimittel- und Futtermittelvormischungen.

Die Erfindung betrifft außerdem ein Verfahren zur Herstellung dieser Wirkstoffzubereitung, das dadurch gekennzeichnet ist, daß der Wirkstoff und der Trägerstoff innig miteinander vermengt werden.

Als grobteilige Getreidekleie kommt bevorzugt Getreidekleie mit einem Korngrößenanteil ≧ 250 µm von mindestens 90 %, insbesondere Hart- und Weichweizengrießkleie wie grobteilige Weizengrießkleie WG 12 in Frage.

Der Begriff Wirkstoff umfaßt Arzneimittel- und Futterzusatzstoffe. Diese sind im Prinzip bekannt und werden beispielsweise beschrieben im Arzneimittelgesetz (§2) und im Futtermittelgesetz (§2).

So können u.a. Coccidiostatika, Anthelmintika, Antibiotika, Mineralstoffe, Vitamine und Leistungsförderer dem Trägerstoff zugesetzt werden. Als Leistungsförderer werden solche Stoffe verstanden, die das Wachstum, den Stoffwechsel, das Körpergewicht, die Gewebezusammensetzung und/oder die Futterverwertung beeinflussen, z. B. werden Sulazuril, Halofuginon, Salinomycinnatrium und Flavophospholipol, insbesondere Sulazuril zugesetzt.

Die nachfolgenden Beispiele dienen zur Erläuterung der Erfindung, insbesondere in ihren bevorzugten Ausführungsformen. Ferner wird die Erfindung durch den Inhalt der Patentansprüche bestimmt. Prozentangaben beziehen sich auf das Gewicht, wenn nichts anderes angegeben wird.

### 1. Untersuchung des Entmischungsverhaltens

Das Entmischungsverhalten wurde geprüft, indem die homogene Vormischung über eine Vibrationsrinne läuft und einen Schüttkegel aufbaut. Aus der Spitze und dem Fuß des Kegels wurden Proben gezogen und analysiert. Abweichungen vom Sollgehalt der homogenen Vormischung sind Indikatoren für eine Entmischung während des Transportes über die Vibrationsrinne oder beim freien Fall.

99 Teile Trägerstoff wurden mit einem Teil Wirkstoff (Sulazuril) in einem Pflugscharmischer gemischt. Nach 5, 10 und 15 min wurden von verschiedenen Stellen des Mischers Proben entnommen und analysiert.

### Gehalt an Wirkstoff Sulazuril (INN):

| Entnahmestelle | 5 mim | | 10 mim | | 15 mim | |
|---|---|---|---|---|---|---|
| | a | b | a | b | a | b |
| 1 | 0.98 | 0.99 | 1.00 | 1.01 | 0.99 | 1.01 |
| 2 | 0.99 | 0.99 | 0.99 | 1.00 | 0.99 | 0.99 |
| 3 | 1.00 | 1.01 | 0.99 | 1.01 | 0.99 | 1.00 |

a) feine Weizengrießkleie (70 % ≦ 250 µm)
b) WG 12

Entmischen nach Transport über Vibrationsrinne:
Vibrationsrinne: Länge 45 cm, Schütthöhe: 15 cm

| Gehalt Wirkstoff: | feine Weizengrießkleie | WG 12 |
|---|---|---|
| Kegelspitze | 1.03 | 1.00 |
| Kegelfuß | 1.02 | 0.99 |

Entmischen nach Stampfen:
250 ml Stampfzylinder, 1250 Hubbewegungen

| Gehalt Wirkstoff | feine Weizengrießkleie | WG 12 |
|---|---|---|
| Oberfläche | 1.03 | 1.01 |
| Boden | 1.02 | 0.99 |

Die Verwendung grober Weizengrießkleie führt weder beim Transport über die Vibrationsrinne noch beim Stampfen zu einem Entmischen der Vormischung.

### 2. Messung der Staubzahl

Die Staubzahl wurde mit einem automatisierten Staubmeßgerät gemessen. Die Meßapparatur besteht aus dem Meßkasten mit einem eingesetzten Staubkasten. Auf dem Kasten ist ein 50 cm langes Fallrohr mit verlängerndem Vorratsrohr und Einfülltrichter aufgesetzt. Das Vorratsrohr ist unten mit einer magnetisch zu öffnenden Klappe vom Fallrohr getrennt. Beim Öffnen wird der gesamte Rohrquerschnitt für 2 sec freigegeben. In dem Meßkasten ist versetzt zur Fallrichtung seitlich eine Beleuchtung (12 V, 15 W) und eine Selenphotozelle (S 60 der Firma Bruno Lange) angebracht, die beide ebenso wie die Magnetklappe durch getrennte Kabel mit dem Meßgerät verbunden werden. Das Meßgerät ist für 220 V Netzspannung ausgelegt und die Spannung auf ± 0,1 % stabilisiert.

Ein Maximalwertspeicher erfaßt bei der Messung den Spitzenwert, speichert und addiert ihn nach 30 sec Meßzeit zu dem Momentanwert. Die Messung ist dann beendet. Die Staubzahl ist die Summe aus dem Spitzenwert und dem Wert nach 30 sec.

Die Staubzahl von feiner Weizengrießkleie betrug: 1,6/1,4; die Staubzahl der groben Weizengrießkleie (WG 12) betrug 0.

### 3. Test auf Verbacken

Die Testmethode zur Bestimmung der Verbackungsneigung ist von Russow et al. in Chem. Ing.-Techn. 40. Jahrgang 1968 / Heft 4, Seite 191 beschrieben.

Der Wert betrug für feine Weizengrießkleie: 4,1 /3,8 kg und für grobe Weizengrießkleie (WG 12) 1,4/2,0 kg (2 Bestimmungen).

## Patentansprüche

1. Wirkstoffzubereitung, dadurch gekennzeichnet, daß sie einen oder mehrere Wirkstoffe und grobteilige Getreidekleie als Trägerstoff enthält.

2. Wirkstoffzubereitung gemäß Anspruch 1, dadurch gekennzeichnet, daß der Trägerstoff aus Weizengrießkleie besteht.

3. Wirkstoffzubereitung gemäß Anspruch 1 und 2, dadurch gekennzeichnet, daß der Trägerstoff aus Weizengrießkleie besteht, die eine Korngrößenverteilung ≧ 250 µm von mindestens 90 % aufweist.

4. Wirkstoffzubereitung gemäß einem oder mehreren der Ansprüche 1 bis 3, dadurch gekennzeichnet, daß ein Wirkstoff aus der Gruppe der Vitamine, Mineralstoffe, Antibiotika, Anthelmintika, Coccidiostatika und Leistungsförderer ausgesucht wird.

5. Wirkstoffzubereitung gemäß einem oder mehreren der Ansprüche 1 bis 4, dadurch gekennzeichnet, daß der Wirkstoff ein Coccidiostatikum ist.

6. Wirkstoffzubereitung gemäß einem oder mehreren der Ansprüche 1 bis 5, dadurch gekennzeichnet, daß als Wirkstoff Sulazuril eingesetzt wird.

7. Wirkstoffzubereitung gemäß einem oder mehreren der Ansprüche 1 bis 6 zur Anwendung bei der Behandlung und Prophylaxe von Parasitosen, insbesondere Coccidiose.

8. Verwendung einer Wirkstoffzubereitung gemäß einem oder mehreren der Ansprüche 1 bis 7 in der Tierernährung und Tiergesundheit, insbesondere als Coccidiostatikum.

9. Verfahren zur Herstellung einer Wirkstoffzubereitung gemäß einem oder mehreren der Ansprüche 1 bis 6, dadurch gekennzeichnet, daß der Trägerstoff mit dem Wirkstoff innig vermengt wird.
